# EUROPEAN PATENT APPLICATION

(11) **EP 2 607 430 A1**
(43) Date of publication of application: **26.06.2013**
(21) Application number: 11818124.7
(22) Date of filing: 11.08.2011
(51) Int. Cl.: C08L 101/00, C08K 5/37, C08L 33/04, C08L 33/26, C08L 71/02

(54) **ANTIBACTERIAL DISPERSION**

(30) Priority: 21.12.2010 JP 2010284772; 19.08.2010 JP 2010183745
(71) Applicant: API Corporation, Chuo-ku, Tokyo 103-0027 (JP)
(72) Inventor: YOSHIMARU, Masaaki, Chikujo-gun Fukuoka 871-0801 (JP)
(74) Representative: HOFFMANN EITLE
(86) International application number: PCT/JP2011/068345
(87) International publication number: WO 2012/023482

(57) **Abstract**

To provide an aqueous antibacterial dispersion which contains a metal pyrithione salt and a cationic polymer and which can maintain a stable dispersed state. An antibacterial dispersion characterized by containing at least one cationic polymer, at least one metal pyrithione salt, and at least one non-anionic surfactant. In an aqueous dispersion of the metal pyrithione salt, the non-anionic surfactant is blended instead of a conventional anionic component, whereby good dispersion stability can be maintained in a cationic polymer blend system.

## Description

### Field of Invention

The present invention relates to an aqueous antibacterial dispersion which contains a metal pyrithione salt and a cationic polymer and which can maintain a stable dispersed state.

### Background of Invention

Polyvalent metal salts of pyrithione (otherwise known as 1-hydroxy-2-pyridinethione, 2-pyridinethiol-1-oxide, 2-pyridinethione, 2-mercaptopyridine-N-oxide, pyridinethione, or pyridinethione-N-oxide) are known as effective biocides and are widely used as anti-foulants, anti-dandruff agents, bactericidal agents, antibacterial agents, and fungicides in applications such as anti-fouling paints, anti-dandruff shampoos, and antibacterial resins. Polyvalent metal salts of pyrithione widely used are zinc pyrithione (ZPT) and copper pyrithione (CPT). Zinc pyrithione is used in hair treatment agents such as anti-dandruff shampoos for the purpose of dandruff prevention. Zinc pyrithione and/or copper pyrithione is used as an anti-fouling component in anti-fouling paints typified by ship-bottom paints.

In general, polyvalent metal salts of pyrithione are sparingly soluble in water. Therefore, for example, zinc pyrithione is often available in the form of an aqueous dispersion in which particulate zinc pyrithione is dispersed in water. Many examples using an anionic dispersant or thickening agent to maintain the dispersion stability are disclosed (for example, Patent Literature 1).

In recent years, a large number of hair treatment agents having both an anti-dandruff effect and a conditioning effect have been developed. In such a hair treatment agent, zinc pyrithione, which serves as an anti-dandruff agent, and a cationic polymer which is a conditioning agent are blended. In the case of producing the hair treatment agent, the cationic polymer is blended with an aqueous dispersion containing an anionic component and zinc pyrithione for the purpose of maintaining dispersion stability and therefore a special technique is necessary for a blending procedure or the like. Thus, it is not necessarily easy for hair treatment agent producers to industrially produce a hair treatment agent with excellent dispersion stability by blending a cationic polymer with a zinc pyrithione dispersion. In an attempt to blend a dispersion containing high concentration of zinc pyrithione with the cationic polymer in advance, simple mixing allows an anionic component in the dispersion to react with the cationic polymer to impair the dispersion performance thereof and therefore zinc pyrithione coagulates or precipitates or an insoluble salt of the anionic component and the cationic polymer precipitates; hence, it has been difficult to produce a stable aqueous dispersion.

The following methods and dispersion have been proposed: methods in which, in order to prevent the re-coagulation of zinc pyrithione in an aqueous dispersion, a water-soluble polymer is ground together with zinc pyrithione such that the surface of zinc pyrithione is coated (Patent Literatures 2 to 5) and an aqueous antibacterial dispersion containing polyvinylpyrrolidone, which is a water-soluble polymer, for the purpose of maintaining the dispersion stability in the case of re-melting after freezing (Patent Literature 6). However, none of these aims or affects the stability of a blend of the cationic polymer.

### List of Documents

### Patent Literature

Patent Literature 1: Korean Patent Publication 2002-009096 A
Patent Literature 2: Japanese Patent Publication 61-152627 A
Patent Literature 3: Japanese Patent Publication 61-151111 A
Patent Literature 4: Japanese Patent Publication 61-57503 A
Patent Literature 5: Japanese Patent Publication 60-224676 A
Patent Literature 6: Japanese Patent 3459833

### Object and Summary of Invention

It is an object of the present invention to provide an aqueous antibacterial dispersion which contains a metal pyrithione salt and a cationic polymer and which can maintain a stable dispersion state.

The inventors have made intensive investigations to solve the above problem. As a result, the inventors have found that the dispersion stability of an aqueous antibacterial dispersion containing a cationic polymer can be maintained well by blending a non-anionic surfactant with an aqueous dispersion containing a metal pyrithione salt, which has been conventionally said to be not necessarily compatible with the cationic polymer, particularly zinc pyrithione.

The present invention has been accomplished on the basis of this finding and is as summarized below.

An antibacterial dispersion according to a first aspect is characterized by containing at least one cationic polymer, at least one metal pyrithione salt, and at least one non-anionic surfactant.

An antibacterial dispersion according to a second aspect is characterized in that in the antibacterial dispersion according to the first aspect, the non-anionic surfactant is a nonionic surfactant and/or an amphoteric surfactant.

An antibacterial dispersion according to a third aspect is characterized in that in the antibacterial dispersion according to the second aspect, the nonionic surfactant contains one or more selected from the group consisting of polyoxyethylene alkyl ethers, polyoxyethylene alkylene derivatives, polyoxyethylene fatty acid esters, polyoxyethylene sorbitan fatty acid esters, polyoxyethylene sorbitol fatty acid esters, polyethylene glycols, alkyl alkanol amides, and nonionic water-soluble polymers.

An antibacterial dispersion according to a fourth aspect is characterized in that in the antibacterial dispersion according to the third aspect, the HLB of the nonionic surfactant ranges from 10 to 20.

An antibacterial dispersion according to a fifth aspect is characterized in that in the antibacterial dispersion according to any one of the first to fourth aspects, the metal pyrithione salt is a salt of any one of Zn, Cu, Fe, Al, and Mg.

An antibacterial dispersion according to a sixth aspect is characterized in that in the antibacterial dispersion according to the fifth aspect, the metal pyrithione salt is a Zn salt.

An antibacterial dispersion according to a seventh aspect is characterized in that in the antibacterial dispersion according to any one of the first to sixth aspects, the cationic polymer is a hair conditioner.

An antibacterial dispersion according to an eighth aspect is characterized in that in the antibacterial dispersion according to the seventh aspect, the cationic polymer is a copolymer which contains constitutional units corresponding to a cationic vinyl monomer (A) represented by the following formula (I) and constitutional units corresponding to nonionic vinyl monomers (B) represented by the following formulae (II) and/or (III), in which the proportion of the constitutional units corresponding to the cationic vinyl monomer (A) to the total constitutional units is 10% to 80% by weight, and which has a weight-average molecular weight of 10,000 to 2,000,000:

CH₂=C (R¹) -CO(O)ₐ- (NH)₁₋ₐ- (CH₂)_{b}-N⁺R²R³R⁴·X⁻ (I)

(where R¹ represents a hydrogen atom or a methyl group; R² and R³ independently represent an alkyl group containing one to 24 carbon atoms, an aryl group containing one to 24 carbon atoms, or an aralkyl group containing one to 24 carbon atoms; R⁴ represents a hydrogen atom, an alkyl group containing one to 24 carbon atoms, an aryl group containing one to 24 carbon atoms, an aralkyl group containing one to 24 carbon atoms, or -CH₂-CH(OH)-CH₂N⁺R⁵R⁶R⁷·Y⁻; R⁵, R⁶, and R⁷ independently represent an alkyl group containing one to 24 carbon atoms, an aryl group containing one to 24 carbon atoms, or an aralkyl group containing one to 24 carbon atoms; X⁻ and Y⁻ independently represent an anion; a represents 0 or 1; and b represents an integer of 1 to 10),

CH₂=C(R⁸)-CO(O)-Z-H (II)

(where R⁸ represents a hydrogen atom or a methyl group and Z represents a bivalent linker containing two or more hydroxyl groups),

CH₂=C (R⁹) -CO-NR¹⁰R¹¹ (III)

(where R⁹ represents a hydrogen atom or a methyl group and R¹⁰ and R¹¹ independently represent a hydrogen atom, an alkyl group containing one to four carbon atoms, or a hydroxyalkyl group containing one to four carbon atoms).

An antibacterial dispersion according to a ninth aspect is characterized in that in the antibacterial dispersion according to any one of the first to eighth aspects, the blending ratio of the pyrithione salt to the cationic polymer is 1/1 to 50/1 on a weight basis.

An antibacterial dispersion according to a tenth aspect is characterized in that in the antibacterial dispersion according to any one of the first to ninth aspects, the content of the metal pyrithione salt is 5% to 70% by weight, the content of the cationic polymer is 0.1% to 40% by weight, and the content of the non-anionic surfactant is 0.01% to 25% by weight.

An antibacterial dispersion according to an eleventh aspect is characterized in that in the antibacterial dispersion according to any one of the first to tenth aspects, the ratio of the content of the metal pyrithione salt to the content of the non-anionic surfactant is such that the pyrithione salt/non-anionic surfactant ratio is 10,000/1 to 1/1 on a weight basis.

An antibacterial dispersion according to a twelfth aspect is characterized in that in the antibacterial dispersion according to any one of the first to eleventh aspects, the antibacterial dispersion according to the twelfth aspect is produced from the following precursor:
(i) a dispersion precursor which is obtained by grinding and mixing a powder of the metal pyrithione salt, the cationic polymer, and water and which has a metal pyrithione salt concentration of 30% to 80% by weight or
(ii) a dispersion precursor which is obtained by grinding and mixing a powder of the metal pyrithione salt, the non-anionic surfactant, the cationic polymer, and water and which has a metal pyrithione salt concentration of 30% to 80% by weight.

### Advantageous Effects of Invention

An antibacterial dispersion which is a metal pyrithione salt-containing antibacterial dispersion of the present invention is one in which a cationic polymer is blended in advance and can maintain high dispersion stability over a long period of time even if a metal pyrithione salt such as zinc pyrithione is contained at high concentration.

Therefore, hair treatment agent producers can produce a hair treatment agent capable of imparting a rinsing/conditioning effect in addition to an anti-dandruff effect without using any special technique in such a manner that an antibacterial dispersion, according to the present invention, containing, for example, zinc pyrithione as a metal pyrithione salt is formulated by adjusting necessary components; hence, the technical burden thereof is significantly reduced. The use of the produced hair treatment agent in combination with a cationic polymer allows the effect of increasing the persistence of zinc pyrithione, which is an anti-dandruff agent, on head hair or scalp to be expected.

Furthermore, an antibacterial dispersion which is a metal pyrithione salt-containing antibacterial dispersion of the present invention can maintain high dispersion stability over a long period of time and therefore can be used for antibacterial fibers, antibacterial nonwoven fabrics, antibacterial clothes, antibacterial resins, antibacterial films, antibacterial paints, agrochemicals, cosmetics, and the like.

### Description of Embodiments

An antibacterial dispersion according to an embodiment of the present invention will now be described in detail.

The antibacterial dispersion of the present invention is characterized by containing at least one cationic polymer, at least one metal pyrithione salt, and at least one non-anionic surfactant.

### [Metal pyrithione salt]

The metal pyrithione salt used in the present invention is not particularly limited. The present invention is particularly effective for polyvalent metal salts of pyrithione (otherwise known as 1-hydroxy-2-pyridinethione, 2-pyridinethiol-1-oxide, 2-pyridinethione, 2-mercaptopyridine-N-oxide, pyridinethione, or pyridinethione-N-oxide), which has low solubility in water and needs to be improved in dispersion stability in water, that is, polyvalent metal pyrithione salts.

Polyvalent metal pyrithione salts preferred for the present invention are Zn (zinc) salts, Cu (copper) salts, Fe (iron) salts, Al (aluminum) salts, Mg (magnesium) salts of pyrithione, and the like. Among these salts, a bis(2-pyridinethiol-1-oxide) zinc salt (zinc pyrithione, which is abbreviated as ZPT), which is useful as an anti-dandruff agent, has excellent effects as a hair treatment agent used in combination with the cationic polymer and therefore is particularly preferred.

One type of metal pyrithione salt only or two or more types of metal pyrithione salt may be contained in the antibacterial dispersion of the present invention.

### [Cationic polymer]

The cationic polymer according to the present invention is not particularly limited and is preferably a general-purpose cationic polymer usually used as a hair treatment agent such as a hair conditioner for rinsing and conditioning effect purposes. Examples of the general-purpose cationic polymer include cationized cellulose generally referred to as Polyquaternium-10, those generally referred to as catigua (cationized guar gum), and the like.

The types and product names of general-purpose cationic polymers blended or used in hair treatment agents for rinsing/conditioning effect purposes are cited below. The cationic polymer used in the present invention is not limited to these polymers in any way.

(1) Polyquaternium-4 (hydroxyethyl cellulose-dimethyl diallylammonium copolymer):
   Celquat L-200, Celquat H-100 (the above are products of Nippon SSC Ltd.), and the like.

(2) Polyquaternium-10 (O-[2-hydroxy-3-(trimethylammonio)propyl]hydroxyethyl cellulose chloride, cationized cellulose):
   Merquat 10 (the above is a product of Nalco Japan Co., Ltd.), Catinal HC-100, Catinal-200, Catinal LC-200 (the above are products of Toho Chemical Industry Co., Ltd.), Leoguard G, Leoguard GP, Leoguard KGP, Leoguard MGP, Leoguard MLP, Leoguard LP (the above are products of Lion Corporation), Celquat SC230M, Celquat 240C (the above are products of Nippon NSC Ltd.), NK Polymer (the above is a product of Nitto Chemical Industry Co.), POIZ C-60M, POIZ C-80M, POIZ C-150L (the above are products of Kao Corporation), Ucare Polymer JR-125, Ucare Polymer JR-30M, Ucare Polymer JR-400 (the above are products of Dow Chemical Japan Ltd./Amerchol Corporation), and the like.

(3) Polyquaternium-6 (polydimethylmethylenepiperidinium chloride):
   Salcare SC30 (the above is a product of Ciba Specialty Chemicals Inc.), Merquat 100 (the above is a product of Nalco Japan Co., Ltd.), Adeka Catioace PD-50 (the above is a product of Adeka Corporation), Cosmuat VG (the above is a product of SENKA Corporation), Lipoflow KY Conc (the above is a product of Lion Corporation), ME Polymer H40W (the above is a product of Toho Chemical Industry Co., Ltd.), and the like.

(4) Polyquaternium-7 (dimethyldiallylammonium chloride-acrylamide copolymer):
   Merquat 2200, Merquat 550, Merquat 740, Merquat S (the above are products of Nalco Japan Co., Ltd.), Lipoflow MN-S, Lipoflow MN (the above are products of Lion Corporation), ME Polymer 09W (the above is a product of Toho Chemical Industry Co., Ltd.), and the like.

(5) Polyquaternium-22 (dimethylallylammonium chloride-acrylic acid copolymer, a solution thereof):
   Merquat 280, Merquat 295 (the above are products of Nalco Japan Co., Ltd.), and the like.

(6) Polyquaternium-24 (O-[2-hydroxy-3-(lauryldimethylammonio)propyl)hydroxyethyl cellulose chloride):
   Quatrisoft Polymer LM-200 (the above is a product of Dow Chemical Japan Ltd./Amerchol Corporation) and the like.

(7) Polyquaternium-47:
   Merquat 2001 (the above is a product of Nalco Japan Co., Ltd.)

(8) Polyquaternium-53:
   Merquat 2003 and Merquat 5300 (the above are products of Nalco Japan Co., Ltd.).

(9) Guar hydroxypropyltrimonium chloride (O-[2-hydroxy-3-(trimethylammonio)propyl] guar gum chloride, cationized guar gum):
   JAGUAR C-13S, JAGUAR 14S, JAGUAR 17 (the above are products of Sansho Co., Ltd./Rodia Inc.), NEOVISCO CS series (the above are products of Sansho Co., Ltd.), Catinal CG-100, Catinal S (the above are products of Toho Chemical Industry Co., Ltd.), and the like.

(10) Cationized Dextran-2 (O-[2-hydroxy-3-(trimethylammonio)propyl] dextran chloride):
   CDC, CDC-L, CDC-H (the above are products of Meito Sangyo. Co., Ltd.), and the like.

(11) Hydroxypropyltrimonium starch chloride (O-[2-hydroxy-3-(trimethylammonio)propyl] starch chloride solution):
   SENSOMER CI-50 (the above is a product of Nalco Japan Co., Ltd.)

Another example of the cationic polymer, which is preferably used in the present invention, is a cationic polymer (this cationic polymer is hereinafter referred to as "cationic polymer A/B" in some cases) that is a copolymer which contains constitutional units corresponding to a cationic vinyl monomer (A) represented by Formula (I) below (that is, constitutional units derived from the cationic vinyl monomer (A) represented by Formula (I)) and constitutional units corresponding to nonionic vinyl monomers (B) represented by Formulae (II) and/or (III) below (that is, constitutional units derived from the nonionic vinyl monomers (B) represented by Formulae (II) and/or (III)), in which the proportion of the constitutional units corresponding to the cationic vinyl monomer (A) to the total constitutional units is 10% to 80% by weight, and which has a weight-average molecular weight of 10,000 to 2,000,000.

CH₂=C(R¹) -CO(O)ₐ- (NH)₁₋ₐ- (CH₂)_{b}-N⁺R²R³R⁴·X⁻ (I)

(where R¹ represents a hydrogen atom or a methyl group; R² and R³ independently represent an alkyl group containing one to 24 carbon atoms, an aryl group containing one to 24 carbon atoms, or an aralkyl group containing one to 24 carbon atoms; R⁴ represents a hydrogen atom, an alkyl group containing one to 24 carbon atoms, an aryl group containing one to 24 carbon atoms, an aralkyl group containing one to 24 carbon atoms, or -CH₂-CH(OH)-CH₂N⁺R⁵R⁶R⁷·Y⁻; R⁵, R⁶, and R⁷ independently represent an alkyl group containing one to 24 carbon atoms, an aryl group containing one to 24 carbon atoms, or an aralkyl group containing one to 24 carbon atoms; X⁻ and Y⁻ independently represent an anion; a represents 0 or 1; and b represents an integer of 1 to 10.)

CH₂=C(R⁸)-CO(O)-Z-H (II)

(where R⁸ represents a hydrogen atom or a methyl group and Z represents a bivalent linker containing two or more hydroxyl groups.)

CH₂=C(R⁹)-CO-NR¹⁰R¹¹ (III)

(where R⁹ represents a hydrogen atom or a methyl group and R¹⁰ and R¹¹ independently represent a hydrogen atom, an alkyl group containing one to four carbon atoms, or a hydroxyalkyl group containing one to four carbon atoms.)

The cationic polymer A/B is described below.

The term "cationic group" as used herein refers to a functional group which contains, for example, a positively charged atom like a nitrogen atom in a quaternary amine and which may be in the form of a salt or may be ionized in a solution. The terms "cationic monomer" and "cationic polymer" refer to a monomer and polymer (including a copolymer), respectively, containing a positively charged atom. The term "nonionic monomer" refers to an electrically neutral monomer containing no charged atom. The term "cationic" used in the terms "cationic group", "cationic polymer", and "cationic monomer" does not include those, so-called "amphoteric", containing both a positively charged atom and a negatively charged atom.
The term "polymer" as used herein includes polymers derived from natural products. The term "(meth)acrylate" is a generic name for methacrylate and acrylate. This applies to (meth)acrylamide, (meth)acryloyl, and (meth)acrylic acid.

The cationic vinyl monomer (A) is not particularly limited and may be a (meth)acrylic quaternary ammonium salt represented by Formula (I). Examples thereof include (meth)acrylic ester quaternary ammonium salts such as N-methacryloyloxyethyl-N,N,N-trimethylammonium chloride and (meth)acrylic amide quaternary ammonium salts such as N-methacryloylaminopropyl-N,N,N-trimethylammonium chloride.

In Formula (I), R¹ is preferably a methyl group; R² and R³ are preferably independently an alkyl group containing one to 24 carbon atoms, more preferably a methyl group or an ethyl group, and particularly preferably a methyl group; R⁴ is preferably an alkyl group containing one to 24 carbon atoms, more preferably a methyl group, an ethyl group, or a butyl group, and particularly preferably a methyl group; R⁵ to R⁷ are preferably independently an alkyl group containing one to 24 carbon atoms, more preferably a methyl group or an ethyl group, and particularly preferably a methyl group; the anions represented by X⁻ and Y⁻ are preferably independently a halogen ion and more preferably a chloride ion, an iodide ion, or a bromide ion; and b is preferably an integer of 1 to 5 and more preferably 2 or 3.

Particular examples of the cationic vinyl monomer (A) include cationic group-containing (meth)acrylic esters such as N-(meth)acryloyloxyethyl-N,N,N-trimethylammonium chloride, N-(meth)acryloyloxyethyl-N-ethyl-N,N-dimethylammonium monoethyl sulfate, N-(meth)acryloyloxyethyl-N,N,N-triethyloxyethylammonium monoethyl sulfate, N-[3-{N'-(meth)acryloyloxyethyl-N',N'-dimethylammonium}-2-hydroxypropyl]-N,N,N-trimethylammonium chloride, and N-[3-{N'-(meth)acryloyloxyethyl-N',N'-diethylammonium}-2-hydroxypropyl]-N,N,N-triethylammonium chloride; cationic group-containing (meth)acrylamides such as N-(meth)acryloylaminopropyl-N,N,N-trimethylammonium chloride, N-(meth)acryloylaminopropyl-N-ethyl-N,N-dimethylammonium monoethyl sulfate, N-(meth)acryloylaminopropyl-N,N-diethyl-N-methylammonium chloride, N-(meth)acryloylaminopropyl-N,N-diethyl-N-methylammonium monomethyl sulfate, N-[3-{N'-(meth)acryloylaminopropyl-N',N'-dimethylammonium}-2-hydroxypropyl]-N,N,N-trimethylammonium chloride, and N-[3-{N'-(meth)acryloylaminopropyl-N',N'-diethylammonium}-2-hydroxypropyl]-N,N,N-trimethylammonium chloride; and the like.

In particular, the cationic vinyl monomer (A) is preferably N-(meth)acryloyloxyethyl-N,N,N-trimethylammonium chloride and N-(meth)acryloylaminopropyl-N,N,N-trimethylammonium chloride and particularly preferably N-(meth)acryloyloxyethyl-N,N,N-trimethylammonium chloride.

The constitutional units corresponding to the cationic vinyl monomer (A) in the cationic polymer A/B are obtained by polymerizing, for example, the cationic vinyl monomer (A) and can also be obtained in such a manner that after a cationic vinyl monomer precursor represented by Formula (IV) below is copolymerized, the copolymer is cationized by carrying out a cationization reaction using a cationizing agent such that the copolymer is converted into a structure having a corresponding cationic group.

CH₂=C (R¹) -CO (O)ₐ- (NH)₁₋ₐ- (CH₂)_{b}-NR²R³ (IV) (where R¹ to R³, a, and b are synonymous with those in Formula (I) and those preferred are also synonymous therewith.)

Examples of the cationic vinyl monomer precursor represented by Formula (IV) include (meth)acrylic esters, such as N-(meth)acryloyloxyethyl-N,N-dimethylamine and N-(meth)acryloyloxyethyl-N,N-diethylamine, having a tertiary amine structure; (meth)acrylamides, such as N-(meth)acryloylaminopropyl-N,N-dimethylamine and N-(meth)acryloylaminopropyl-N,N-diethylamine, having a tertiary amine structure; and the like.

Examples of the cationizing agent include alkyl halides such as methyl chloride, cationic group-containing cationizing agents such as 3-chloro-2-hydroxypropyl-N,N,N-trimethylammonium chloride, and the like. The cationization reaction can be carried out, for example, at 20°C to 100°C for 1 to 20 hours by adding the cationizing agent to a solution of the copolymer.

One type cationic vinyl monomer (A) or a precursor thereof may be used alone or two or more types thereof may be used in combination.

The content of the constitutional units corresponding to the cationic vinyl monomer (A) in the cationic polymer A/B is preferably 10% by weight or more, more preferably 20% by weight or more, and further more preferably 25% by weight or more. The content thereof is preferably 80% by weight or less, more preferably 70% by weight or less, and further more preferably 60% by weight or less. When being used in a hair treatment agent in combination with an anionic surfactant, the constitutional units corresponding to the cationic vinyl monomer (A) probably form a complex therewith to allow the cationic polymer A/B to be likely to adhere to hair. When the content of the constitutional units corresponding to the cationic vinyl monomer (A) is 10% by weight or more, the complex can be sufficiently formed with the anionic surfactant and the adhesion to hair or the like can be sufficiently maintained. When the content thereof is 80% by weight or less, the cationic polymer A/B can be blended with the hair treatment agent without coagulation, which is preferred.

The nonionic vinyl monomers (B) are not particularly limited and may be represented by Formula (II) or (III). The nonionic vinyl monomers (B) have the effect of imparting hydrophilicity to the cationic polymer A/B because of hydrophilicity due to the nonionic vinyl monomers (B). This increases the water solubility of the cationic polymer A/B and probably maintains the water solubility of the cationic polymer A/B when the cationic polymer A/B forms a complex with a surfactant.

The nonionic vinyl monomer (B) represented by Formula (II) (hereinafter referred to as "nonionic vinyl monomer (B-II)" in some cases) is not particularly limited and may be one included in this formula and Z is preferably a bivalent linker which contains two to nine carbon atoms and two or more hydroxyl groups and which may contain an oxygen atom and/or a nitrogen atom. More preferably, Z is a bivalent linker which contains two or more hydroxyl groups and which is represented by {-(CH₂)_{c}-(CR¹²R¹³)_{d}-(CHOH)ₑ-(CH₂O)_{f}-}, where R¹² and R¹³ independently represent an alkyl group containing one to three carbon atoms or a hydroxyalkyl group containing one to three carbon atoms, c is an integer of 1 to 4, d is 0 or 1, e is an integer of 1 to 6, and f is an integer of 0 to 2. Herein, the order of the four linking groups -(CH₂)-, - (CR¹²R¹³)-, -(CHOH)-, and - (CH₂O) - in the braces {} is arbitrary and may be random. For example, when c is 2, d is 0, e is 1, and f is 0, a structure in the braces {} may be {-(CH₂)-(CH₂)-(CHOH)-} or {-(CH2)-(CHOH)-(CH2)-}. In particular, Z is preferably a bivalent linker which contains two or more hydroxyl groups and which is represented by {-(CH₂)_{c}-(CHOH)ₑ-}, {-(CH₂)_{c}-(CR¹²R¹³)-(CHOH)-}, or {-[(CH₂)-(CHOH)-(CH_{2O}))_{g}-}, where R¹², R¹³, c, and e are the same as the above and g is an integer of 1 to 3.

Examples of the nonionic vinyl monomer (B-II) include 2,3-dihydroxypropyl (meth)acrylate, 2,3,4-trihydroxybutyl (meth)acrylate, 2,3,4,5,6-pentahydroxyhexyl (meth)acrylate, pentaerythritol (meth)acrylate, diglycerin (meth)acrylate, triglycerin (meth)acrylate, and the like. The nonionic vinyl monomer (B-II) is most preferably 2,3-dihydroxypropyl (meth)acrylate.

The nonionic vinyl monomer (B) represented by Formula (III) (hereinafter referred to as "nonionic vinyl monomer (B-III)" in some cases) is not particularly limited and may be one included in this formula and R⁹ is preferably a hydrogen atom. Examples of the alkyl or hydroxyalkyl group, represented by R¹⁰ or R¹¹, containing one to four carbon atoms include a methyl group, an ethyl group, a propyl group, an isopropyl group, a butyl group, an isobutyl group, a hydroxymethyl group, a 2-hydroxyethyl group, and the like. The sum of the number of carbon atoms in R¹⁰ and R¹¹ is preferably 2 to 4 and most preferably 2.

Examples of the nonionic vinyl monomer (B-III) include alkylacrylamides such as N-methyl(meth)acrylamide, N-ethyl(meth)acrylamide, N-propyl(meth)acrylamide, and N-isopropyl(meth)acrylamide; dialkylacrylamides such as N,N-dimethyl(meth)acrylamide and N,N-diethyl(meth)acrylamide; hydroxyalkyl(meth)acrylamides such as N-hydroxymethyl(meth)acrylamide, N-(2-hydroxyethyl)(meth)acrylamide, N,N-bis(hydroxymethyl)(meth)acrylamide, and N,N-bis(2-hydroxyethyl)(meth)acrylamide; and the like. In particular, N-(2-hydroxyethyl)(meth)acrylamide is preferred.

The nonionic vinyl monomers (B) may be used alone or in combination. In particular, two or more types of either nonionic vinyl monomer (B-II), which is represented by Formula (II), or nonionic vinyl monomer (B-III), which is represented by Formula (III), may be used in combination or one or more types of nonionic vinyl monomer (B-II) and one or more types of nonionic vinyl monomer (B-III) may be used in combination. One or more types of nonionic vinyl monomer (B-II) and one or more types of nonionic vinyl monomer (B-III) are preferably used in combination because the persistence of the metal pyrithione salt in hair treatment agents is increased.

The content of the constitutional units corresponding to the nonionic vinyl monomer (B) in the cationic polymer A/B is preferably 20% by weight or more, more preferably 30% by weight or more, and further more preferably 40% by weight or more. The content thereof is preferably 90% by weight or less, more preferably 80% by weight or less, and further more preferably 75% by weight or less. When the content of the constitutional units corresponding to the nonionic vinyl monomer (B) is 20% by weight or more, the cationic polymer A/B has increased water solubility and can sufficiently maintain water solubility even if the cationic polymer A/B and, for example, an anionic surfactant form a complex. Adjusting the content thereof to 90% by weight or less is preferable in maintaining the complex of the anionic surfactant.

The cationic polymer A/B may further contain constitutional units derived from another vinyl monomer. However, when an anionic functional group is present in the cationic polymer A/B, the anionic functional group may possibly hinder the formation of a complex particularly in the case of using the anionic surfactant. Therefore, those containing a small number of anionic functional groups (for example, 10% by mole or less of the total of functional groups) are preferred and those containing substantially no anionic functional groups are more preferred. The term "those containing substantially no" as used herein refers to those exhibiting no anionicity at, for example, a pH of 3 to 8.

Examples of this vinyl monomer include nonionic monomers such as esters of (meth)acrylic acid with alcohols containing one to 22 carbon atoms, amides of (meth)acrylic acid with alkylamines containing one to 22 carbon atoms, monoesters of (meth)acrylic acid with ethylene glycol, 1,3-propylene glycol, and the like, esters in which hydroxyl groups are etherified with methanol, ethanol, or the like, (meth)acryloylmorpholine, and hydroxylmethylacrylamide; amino acid-type cationic monomers such as diallyl-type quaternary ammonium salts including N,N-dimethyl-N,N-diallylammonium chloride and products of the reaction of L-arginine with glycidyl methacrylate; amphoteric monomers such as betaine group-containing (meth)acrylic esters and betaine group-containing (meth)acrylamides; semi-polar monomers such as amine oxide group-containing (meth)acrylic esters and amine oxide group-containing (meth)acrylamides; and the like.

The content of constitutional units derived from this vinyl monomer in the cationic polymer A/B can be appropriately determined without departing from the scope of the present invention. The content thereof can be appropriately determined within such a range that, for example, solubility as a water-soluble resin or a conditioning effect as used in hair treatment agents is not impaired. In general, the content in the cationic polymer A/B is preferably 30% by weight or less and more preferably 20% by weight or less.

The content of the constitutional units corresponding to the cationic vinyl monomer (A), the constitutional units corresponding to the nonionic vinyl monomers (B), and the constitutional units derived from this vinyl monomer in the cationic polymer A/B can be determined from the amount of each monomer used to produce the cationic polymer A/B and can be measured by the IR absorption of a hydroxyl group or an amide group, the ¹H-NMR of a methyl group close to a cationic group, or the ¹³C-NMR thereof.

The cationic polymer A/B contains the constitutional units corresponding to the cationic vinyl monomer (A) and therefore contains cationic groups. The cationic polymer A/B contains the constitutional units corresponding to the nonionic vinyl monomer (B-II), which contains a hydroxyl group, and/or the constitutional units corresponding to the nonionic vinyl monomer (B-III), which contains a hydroxyalkyl group or an amide group in some cases, and therefore contains a functional group, that is, at least one of the hydroxyalkyl group or the amide group containing a nonionic substituent only.

The upper limit of the amount of the cationic groups, which are contained in the cationic polymer A/B, is usually 10.0 meq/g and preferably 5.0 meq/g. The lower limit thereof is usually 0.1 meq/g and preferably 0.4 meq/g. When the amount of the cationic groups is 10.0 meq/g or less, the compatibility is good because aggregates are scarcely formed when blended with a hair treatment agent. When the amount thereof is 0.1 meq/g or more, the adhesion to hair is good when used as a hair treatment agent.

The upper limit of the amount of the hydroxyl group, which is contained in the cationic polymer A/B, is usually 20.0 meq/g, preferably 15.0 meq/g, and particularly preferably 10.0 meq/g. The lower limit thereof is usually 1.5 meq/g, preferably 2.0 meq/g, and particularly preferably 2.5 meq/g. The upper limit of the amount of the amide group containing the nonionic substituent only is usually 10.0 meq/g, preferably 8.0 meq/g, and more preferably 6.0 meq/g. The lower limit thereof is usually 1.5 meq/g, preferably 2.0 meq/g, and more preferably 2.5 meq/g.

The cationic polymer A/B satisfies at least one of the amount of the hydroxyl group and the amount of the amide group containing the nonionic substituent only. However, when the cationic polymer A/B does not contain the amide group containing the nonionic substituent only, the amount of the hydroxyl group is preferably 4.2 meq/g or more in order to enhance the effect of adsorbing the metal pyrithione salt. When the amount of the hydroxyl group is 20.0 meq/g or less, the cationic polymer A/B has reduced hydrophilicity and therefore precipitates are likely to be formed during rinsing. When the amount of the hydroxyl group 1.5 meq/g or more or the amount of the amide group containing the nonionic substituent only is 1.5 meq/g or more, the adsorption of the metal pyrithione salt is good.

Thus, it is preferred that the cationic polymer A/B contains at least one of the hydroxyl group and the amide group containing the nonionic substituent only, the amount of the hydroxyl group is 1.5 meq/g to 20.0 meq/g, the amount of the amide group containing the nonionic substituent only 1.5 meq/g to 10.0 meq/g, and the amount of the cationic groups is 0.1 meq/g to 10 meq/g.
Herein, the amount of each functional group represents the milligram equivalent of the functional group per g of the cationic polymer A/B, can be determined or analyzed by various methods, and it is convenient to regard the amount of the functional group as the amount of charge during the synthesis of the cationic polymer A/B. This way is used in examples below.

The cationic polymer A/B used in the present invention preferably has such water solubility that an aqueous solution with a concentration of 5% by weight or more can be formed at room temperature, that is, 25°C. That is, the cationic polymer A/B is preferably such a water-soluble resin that the transmittance of an aqueous solution with a concentration of 5% by weight or more is 80% or more at a wavelength of 550 nm with respect to water and the aqueous solution is stable. The cationic polymer A/B is more preferably one capable of forming an aqueous solution with a concentration of 20% by weight or more.

The cationic polymer A/B used in the present invention can be produced in such a manner that, for example, monomers providing the respective constitutional units or precursors thereof are mixed together and are copolymerized by a polymerization reaction such as solution polymerization, suspension polymerization, or emulsion polymerization, followed by a cationization reaction as required.

The polymerization reaction is preferably carried out in water and/or a hydrophilic organic solvent. Examples of the hydrophilic organic solvent include ketone solvents such as acetone, methyl ethyl ketone, and methyl isobutyl ketone; alcohol solvents such as methanol, ethanol, n-propanol, i-propanol, n-butanol, i-butanol, and sec-butanol; and the like. The water and/or the hydrophilic organic solvent may be used alone or in combination. In particular, an alcohol solvent and/or water is preferably used.

A polymerization initiator may be used in the polymerization reaction. Usable examples of the polymerization initiator include, but are not limited to, azo compounds such as 2,2'-azobisisobutyronitrile, 2,2'-azobis(2-methyl-N-(2-hydroxyethyl)-priopionamide), 2,2'-azobis(2,4-dimethylvaleronitrile), 2,2'-azobis(4-methoxy-2,4-dimethylvaleronitrile), dimethyl-2,2'-azobisisobutyrate, 2,2'-azobis(2-methylbutyronitrile), 1,1'-azobis(1-cyclohexanecarbonitrile), 2,2'-azobis(2-methyl-N-(2-hydroxyethyl)-propionamide), and 2,2'-azobis(2-amidinopropane) dihydrochloride; peroxides such as benzoyl peroxide, dicumyl peroxide, di-t-butyl peroxide, lauroyl peroxide; persulfates; redox systems; and the like. The polymerization initiator is preferably used in the range of 0.01% to 5% by weight with respect to the total of monomers used in reaction and more preferably 0.1% to 3% by weight.

The polymerization reaction can be carried out in, for example, an inert atmosphere such as nitrogen or argon for usually one to 30 hours at preferably 30°C to 120°C and more preferably 40°C to 100°C. After the end of the polymerization reaction, a produced copolymer is preferably isolated from a reaction solution by appropriate procedure such as evaporating a solvent or the addition of a less soluble solvent. The cationic polymer A/B obtained in this way can be used to produce the antibacterial dispersion of the present invention directly or after being purified. Purification can be performed using appropriate procedure such as re-precipitation, solvent washing, and membrane separation alone or in combination.

Usable examples of the cationic polymer A/B used in the present invention include, but are not limited to, those, such as random copolymers, block copolymers, and graft copolymers, having an arbitrary structure. A random copolymer can be synthesized in such a manner that all monomers used for polymerization are mixed and are reacted at once by adding an initiator thereto or in such a manner that some or all of the monomers are reacted gradually while being added. The monomers can be polymerized in such a manner that the monomers are added while being varied in composition. A block copolymer can be synthesized using a known radical initiator or catalyst. A graft copolymer can be synthesized by a method using macromers containing vinyl groups, a method in which polymers containing reactive functional groups are reacted, or the like.

In particular, those capable of being synthesized from inexpensive raw materials without needing special steps are industrially useful and are preferred and random copolymers which can be most readily produced using common raw materials are most preferred.

In the case of blending the cationic polymer A/B in the antibacterial dispersion, several types of copolymer can be used in combination. In this case, the cationic polymer A/B can be blended in such a manner that the cationic polymer A/B is separately synthesized and is then mixed. Alternatively, a mixture of two types of cationic polymer A/B can be obtained in such a manner that a type of cationic polymer A/B is produced in advance and a monomer component forming another type of cationic polymer A/B is added to a reaction solution thereof and is polymerized. Likewise, a mixture of more types of cationic polymer A/B can be obtained by repeating the addition and polymerization of the monomer component. In the present invention, the latter method, which can be readily produced, is preferred.

The cationic polymer A/B preferably has a weight-average molecular weight of 10,000 to 2,000,000. When the weight-average molecular weight thereof is 10,000 or more, a polyvalent metal pyrithione salt can be stably dispersed in the antibacterial dispersion. The weight-average molecular weight of the cationic polymer A/B is more preferably 100,000 or more and further more preferably 200,000 or more. When the weight-average molecular weight of the cationic polymer A/B is 2,000,000 or less, the solution viscosity of the cationic polymer A/B can be appropriately maintained, the compatibility is enhanced, handling is easy in production. The weight-average molecular weight of the cationic polymer A/B is more preferably 1,000,000 or less and further more preferably 700,000 or less.
The weight-average molecular weight of the cationic polymer A/B can be measured by gel permeation chromatography (for example, water/methanol/acetic acid/sodium acetate is used as an eluent) and can be determined using polyethylene glycol as a standard substance.

The solution viscosity of the cationic polymer A/B is preferably within an appropriate range and is preferably such a level that an aqueous solution having a concentration of 20% by weight or more and low viscosity can be prepared. The viscosity of a 20% by weight aqueous solution of the cationic polymer A/B is preferably 100,000 mPa·s or less, more preferably 50,000 mPa·s or less, further more preferably 20,000 mPa·s or less, and most preferably 5,000 mPa·s or less at 25°C. The viscosity thereof is usually 10 mPa·s or more. This is because in the case of blending the cationic polymer A/B in the antibacterial dispersion at high concentration, the concentration of an antibacterial component is not excessively diluted and a high-concentration antibacterial dispersion can be prepared so as not to have needlessly high viscosity, which results in that a product which is the antibacterial dispersion is readily transported and it is preferred that a high-concentration, low-viscosity solution can be obtained.

The viscosity can be adjusted by controlling, for example, the degree of polymerization of the cationic polymer A/B. Furthermore, the molecular weight and viscosity can be controlled by adding a cross-linking agent such as a polyfunctional acrylate or adjusting the amount of the added cross-linking agent. However, there is a problem in that the control of the cross-linking agent is difficult for industrial production because the slightly excessive addition of the cross-linking agent causes significant increases in molecular weight and viscosity. Excessive increases in molecular weight and viscosity cause less compatibility in some cases as described above and therefore are not preferred. The amount of the added cross-linking agent is preferably controlled within such a range that the molecular weight is not increased during polymerization. The amount thereof is preferably, for example, 0.1% by weight or less of the amount of the cationic polymer A/B and more preferably 0.01% by weight or less thereof. It is most preferred that the cross-linking agent is not used for the polymerization of the cationic polymer A/B and is not contained as a constitutional component of the cationic polymer A/B.

An example of a method for controlling the molecular weight of the cationic polymer A/B is a method using a chain transfer agent for polymerization. The addition of the chain transfer agent allows a reduction in molecular weight. However, there is a problem in that the control thereof is difficult for industrial production because the excessive amount of the added chain transfer agent causes an excessive reduction in molecular weight. For example, thiols, such as thioglycol, alkyl thiols, and thioglycolic acid, usually used as chain transfer agents have a disadvantage causing environmental pollution by their bad smell. Thus, it is preferred that no chain transfer agent is used.

### [Non-anionic surfactant]

The non-anionic surfactant used in the present invention functions as a wetting dispersant, emulsifying agent, or thickening agent for antibacterial dispersions and is one other than anionic surfactants, that is, a nonionic, amphoteric, or cationic surfactant or a special surfactant such as a fluorine-containing surfactant or a silicone-based surfactant including polyether-modified silicone. The non-anionic surfactant is preferably a nonionic or amphoteric surfactant and more preferably a nonionic surfactant from the viewpoint of the compatibility with the cationic polymer and a dispersion-stabilizing effect. The surfactant is usually water-soluble and preferably has a molecular weight of about several tens to 150 thousand and more preferably about several hundreds to 100 thousand.

Preferable examples of the nonionic surfactant include polyoxyethylene alkyl ethers; polyoxyethylene alkylene derivatives; polyoxyethylene fatty acid esters; polyoxyethylene sorbitan fatty acid esters; polyoxyethylene sorbitol fatty acid esters; polyethylene glycols; alkyl alkanol amides; polyoxyethylene/polyoxypropylene copolymers; polyvinylpyrrolidones; polyvinyl alcohols; and nonionic water-soluble polymers that are water-soluble cellulose derivatives such as hydroxyethyl cellulose, hydroxypropyl cellulose, hydroxypropyl methyl cellulose, and methyl cellulose.
The alkyl alkanol amides are preferably coconut fatty acid diethanolamide (cocamide DEA), coconut fatty acid monoethanolamide (cocamide MEA), lauric acid diethanolamide (lauramide DEA), coconut fatty acid N-methylethanolamide, and the like.
Particularly preferred are the nonionic surfactants having an HLB of 10 to 20, such as polyoxyethylene alkylene derivatives, polyoxyethylene fatty acid esters, polyoxyethylene sorbitan fatty acid esters, polyoxyethylene sorbitol fatty acid esters, polyethylene glycols, alkyl alkanol amides, polyoxyethylene/polyoxypropylene copolymers, polyvinylpyrrolidones, polyvinyl alcohols, and nonionic water-soluble polymers that are water-soluble cellulose derivatives such as hydroxyethyl cellulose. Especially preferred are polyoxyethylene alkyl ethers, polyoxyethylene alkylene derivatives, polyoxyethylene fatty acid esters, polyoxyethylene sorbitan fatty acid esters, polyethylene glycols, polyvinylpyrrolidones, polyvinyl alcohols, and hydroxyethyl cellulose, having an HLB of 10 to 20.
The amphoteric surfactant is preferably an amphoteric acrylic resin or lauryl hydroxysulfobetaine.

The nonionic surfactant preferably has an HLB of 10 or more because the nonionic surfactant usually forms hydrophilic micelles containing hydrophilic groups directed outward to exhibit surface-activating actions such as O/W type emulsification, the wetting dispersion of water systems, and the solubilization thereof. The upper limit of HLB is 20 by definition. Thus, the HLB of the nonionic surfactant is preferably 10 to 20.

The product names of nonionic surfactants usable in the present invention are cited below. The nonionic surfactant used in the present invention is not limited to these surfactants in any way.

(Polyoxyethylene alkyl ethers)
"EMULGEN 420" (polyoxyethylene (13) oleyl ether, HLB = 13.6) produced by Kao Corporation
"PEGNOL O-16A" (polyoxyethylene oleyl ether, HLB = 14.5) produced by Toho Chemical Industry Co., Ltd.
"EMULGEN 409V" (polyoxyethylene (9) oleyl ether, HLB = 12.0) produced by Kao Corporation
"EMULGEN 109P" (polyoxyethylene (9) lauryl ether, HLB = 13.6) produced by Kao Corporation
"EMULGEN 123P" (polyoxyethylene (23) lauryl ether, HLB = 16.9) produced by Kao Corporation
"EMULGEN 709" (polyoxyethylene higher alcohol ether, HLB = 13.3) produced by Kao Corporation
"EMULGEN 1135S-70" (polyoxyethylene alkyl ether, HLB = 17.9) produced by Kao Corporation

(Polyoxyethylene alkylene derivatives)
"EMULGEN A-60" (polyoxyethylene distyrylphenyl ether, HLB 12.8) produced by Kao Corporation
"NOIGEN EA-137" (same as above) produced by Dai-ichi Kogyo Seiyaku Co., Ltd.

### (Polyoxyethylene fatty acid eaters)

"EMANON 3199V" (polyoxyethylene monostearate, HLB = 19.4) produced by Kao Corporation
"EMANON 4110" (polyoxyethylene (10) monooleate, HLB = 11.6) produced by Kao Corporation
"EMANON 1112" (polyoxyethylene (12) monolaurate, HLB = 13.7) produced by Kao Corporation

(Polyoxyethylene sorbitan fatty acid esters)
"IONET T-80V" (polyoxyethylene (20) sorbitan monooleate, HLB = 15.0) produced by Sanyo Chemical Industries, Ltd.
"SOLGEN TW-80" (same as above) produced by Dai-ichi Kogyo Seiyaku Co., Ltd.
"RHEODOL TW-O120" (same as above) produced by Kao Corporation
"RHEODOL TW-L120" (polyoxyethylene (20) sorbitan monolaurate, HLB = 16.7) produced by Kao Corporation

(Polyoxyethylene sorbitol fatty acid ester)
"RHEODOL 460V" (polyoxyethylene sorbit tetraoleate (60 E. O.), HLB = 13.8) produced by Kao Corporation

(Polyethylene glycols)
"PEG 1000" (Polyethylene Glycol 1000) produced by Wako Pure Chemical Industries, Ltd.
"Toho Polyethylene Glycol 1000" (same as above) produced by Toho Chemical Industry Co., Ltd.
"Toho Polyethylene Glycol 6000" (Polyethylene Glycol 6000) produced by Toho Chemical Industry Co., Ltd.
"Toho Polyethylene Glycol 1500" (Polyethylene Glycol 1500) produced by Toho Chemical Industry Co., Ltd.
"PEG 1540" (Polyethylene Glycol 1540) produced by Dai-ichi Kogyo Seiyaku Co., Ltd.
"PEG 2000" (Polyethylene Glycol 2000) produced by Dai-ichi Kogyo Seiyaku Co., Ltd.
"PEG 4000" (Polyethylene Glycol 4000) produced by Dai-ichi Kogyo Seiyaku Co., Ltd.
"PEG 6000" (Polyethylene Glycol 6000) produced by Dai-ichi Kogyo Seiyaku Co., Ltd.

(Alkyl alkanol amides)
· Coconut fatty acid diethanolamide (cocamide DEA) "DIANOL CDE" produced by Dai-ichi Kogyo Seiyaku Co., Ltd.
   "TOHOL N-220XM" produced by Toho Chemical Industry Co., Ltd.
· Coconut fatty acid monoethanolamide (cocamide MEA)
   "DIANOL CME" produced by Dai-ichi Kogyo Seiyaku Co., Ltd.
   "TOHOL N-120" produced by Toho Chemical Industry Co., Ltd.
· Lauric diethanolamide (lauramide DEA)
   "AMINON L-02" produced by Kao Corporation
   "TOHOL N-230X" produced by Toho Chemical Industry Co., Ltd.
· Coconut fatty acid N-methylethanolamide
   "AMINON C-11S" produced by Kao Corporation

(Polyoxyethylene/polyoxypropylene copolymers)
"EMULGEN PP-290" (polyoxyethylene/polyoxypropylene = 160/30, copolymer) produced by Kao Corporation
"EPAN 680" (same as above) produced by Dai-ichi Kogyo Seiyaku Co., Ltd.

(Polyvinylpyrrolidones)
"PVP K15", "PVP K30", "PVP K60", "PVP K90", and "PVP K120" produced by ISP
"PVP K15", "PVP K30", "PVP K60", and "PVP K90" produced by Dai-ichi Kogyo Seiyaku Co., Ltd.

(Polyvinyl alcohols)
"KURARAY POVAL PVA-120" produced by Kuraray Co., Ltd.
"GOHSENOL NL-05" produced by The Nippon Synthetic Chemical Industry Co., Ltd.
"GOHSENOL GL-03" produced by The Nippon Synthetic Chemical Industry Co., Ltd.

### (Hydroxyethyl cellulose)

"HEC" (hydroxyethyl cellulose) produced by Wako Pure Chemical Industries, Ltd.
"HEC Daicel SE 400" produced by Daicel FineChem Ltd.
"HEC Daicel SE 500" produced by Daicel FineChem Ltd.
"HEC Daicel SE 600" produced by Daicel FineChem Ltd.
"HEC Daicel SE 900" produced by Daicel FineChem Ltd.

### (Amphoteric)

"YUKAFORMER 204W" (amphoteric acrylic resin) produced by Mitsubishi Chemical Corporation
"AMPHITOL 20HD" (lauryl hydroxysulfobetaine) produced by Kao Corporation

One type of non-anionic surfactant, such as the nonionic surfactant, only or two more types of non-anionic surfactant may be contained in the antibacterial dispersion of the present invention.
In particular, when two or more selected from the group consisting of polyoxyethylene alkyl ethers, polyoxyethylene sorbitan fatty acid esters, polyethylene glycols, and hydroxyethyl cellulose, having an HLB of 10 to 20 are contained therein, the effect of suppressing the coagulation of the metal pyrithione salt and the effect of preventing the precipitation thereof are excellent.

### [Blending ratio]

The content of the metal pyrithione salt in the antibacterial dispersion of the present invention is preferably 5% by weight or more and more preferably 10% by weight or more. The content thereof is preferably 70% by weight or less, more preferably 60% by weight or less, and particularly preferably 50% by weight or less.
When the content of the metal pyrithione salt is not less than the above lower limit, the amount of the metal pyrithione salt that the antibacterial dispersion needs can be met. When the content thereof is not more than the above upper limit, dispersion stability or functionality due to another component can be obtained.

The content of the cationic polymer in the antibacterial dispersion of the present invention is preferably 0.1% by weight or more, more preferably 0.5% by weight or more, and particularly preferably 1% by weight or more. The content thereof is preferably 40% by weight or less, more preferably 30% by weight or less, and particularly preferably 25% by weight or less.
When the content of the cationic polymer is not less than the above lower limit, functionality due to blending the cationic polymer can be obtained. When the content thereof is not more than the above upper limit, the contents of the metal pyrithione salt and the non-anionic surfactant are sufficient and the antibacterial dispersion is allowed to have excellent dispersion stability and antibacterial activity.

The content of the non-anionic surfactant in the antibacterial dispersion of the present invention is preferably 0.01% by weight or more, more preferably 0.05% by weight or more, and particularly preferably 0.1% by weight or more. The content thereof is preferably 25% by weight or less, more preferably 15% by weight or less, and particularly preferably 10% by weight or less.
When the content of the non-anionic surfactant is not less than the above lower limit, the effect of enhancing dispersion stability due to blending the non-anionic surfactant can be sufficiently obtained. When the content thereof is not more than the above upper limit, the contents of the metal pyrithione salt and the non-anionic surfactant are sufficient and the antibacterial dispersion is allowed to have excellent antibacterial activity and functionality.

The ratio of the content of the metal pyrithione salt to the content of the cationic polymer in the antibacterial dispersion of the present invention is such that the pyrithione salt/cationic polymer ratio is preferably 1/1 to 50/1, more preferably 1/1 to 25/1, and particularly preferably 1/1 to 10/1 on a weight basis.

The ratio of the content of the metal pyrithione salt to the content of the non-anionic surfactant in the antibacterial dispersion of the present invention is such that the pyrithione salt/non-anionic surfactant ratio is preferably 10,000/1 to 1/1, more preferably 1,000/1 to 2/1, and particularly preferably 100/1 to 3/1 on a weight basis. When the pyrithione salt/non-anionic surfactant ratio is within this range, good dispersion stability can be obtained.

The antibacterial dispersion of the present invention is generally an aqueous dispersion which preferably contains the metal pyrithione salt, the cationic polymer, and the remainder excluding the non-anionic surfactant is water. The content of water in the antibacterial dispersion of the present invention is usually about 10% to 80% by weight.

The antibacterial dispersion of the present invention may contain components other than the metal pyrithione salt, the cationic polymer, and the non-anionic surfactant, that is, for example, an antibacterial component (for example, an antiseptic) other than the metal pyrithione salt, an inorganic salt such as NaCl, a pH adjuster, a perfume, an anionic surfactant, and the like within a range not impairing an object of the present invention.

### [Production method]

A method for producing the antibacterial dispersion of the present invention is not particularly limited. In the case of using a powder of the metal pyrithione salt, the following method is cited: a method in which the antibacterial dispersion is obtained in such a manner that a dispersion precursor having a metal pyrithione salt concentration of about 30% to 80% by weight is obtained by grinding and mixing the metal pyrithione salt, the cationic polymer, and water; the cationic polymer and the non-anionic surfactant are further added to the dispersion precursor as required, followed by mixing; and the concentration of the mixture is adjusted by adding water thereto as required.
Alternatively, the following method is cited: a method in which the antibacterial dispersion is obtained in such a manner that a dispersion precursor having a metal pyrithione salt concentration of about 30% to 80% by weight is obtained by grinding and mixing the metal pyrithione salt, the non-anionic surfactant, the cationic polymer, and water; the cationic polymer and the non-anionic surfactant are further added to the dispersion precursor as required, followed by mixing; and the concentration of the mixture is adjusted by adding water thereto as required.

In the case of supplying the metal pyrithione salt in the form of an aqueous dispersion, predetermined amounts of the non-anionic surfactant and the cationic polymer may be added to the aqueous dispersion of the metal pyrithione salt, followed by concentration adjustment by further adding water thereto as required and then mixing.

The metal pyrithione salt, which is contained in the antibacterial dispersion of the present invention, preferably has a D₅₀ of about 0.1 µm to 5 µm and more preferably about 0.1 µm to 2 µm as measured by a method described in the term "EXAMPLES" below.

### EXAMPLES

The present invention is further described below in detail with reference to production examples, examples, and comparative examples. The present invention is not limited to examples below in any way.

Details of raw materials used are as described below.

### [Metal pyrithione salts]

ZPT-100: "TOMICIDE ZPT-100" (powdery ZPT) produced by API Corporation
ZPT-50: "TOMICIDE ZPT-50" (a 50 weight percent aqueous dispersion of ZPT, D₅₀ = 0.2-0.4 µm) produced by API Corporation

### [Cationic polymer]

Cationic polymer A/B: an aqueous solution of a cationic polymer A/B produced in Production Example 1 (a solid concentration of 40% by weight)

### [Surfactants]

EMULGEN 420: polyoxyethylene (13) oleyl ether produced by Kao Corporation, HLB = 13.6
IONET T-80V: polyoxyethylene (20) sorbitan monooleate produced by Sanyo Chemical Industries, Ltd., HLB = 15.0
YUKAFORMER 204W: an amphoteric acrylic resin (a solid content of 17% by weight) produced by Mitsubishi Chemical Corporation
PVP K30: polyvinylpyrrolidone produced by ISP, a K-value of 26 to 34
EMANON 3199V: polyoxyethylene (12) monolaureate produced by Kao Corporation, HLB = 19.4
EMANON 4110: polyoxyethylene (10) monooleate produced by Kao Corporation, HLB = 11.6
HEC: hydroxyethyl cellulose produced by Wako Pure Chemical Industries, Ltd.
SE 600: HEC Daicel SE 600 produced by Daicel FineChem Ltd.
PEG 1000: Polyethylene Glycol 1000 produced by Wako Pure Chemical Industries, Ltd.
PEG 6000: Polyethylene Glycol 6000 produced by Dai-ichi Kogyo Seiyaku Co., Ltd.
DIANOL CDE: coconut fatty acid diethanolamide (cocamide DEA) produced by Dai-ichi Kogyo Seiyaku Co., Ltd.

### Production Example 1: Production of cationic polymer A/B

Into a reactor equipped with a reflux condenser, a dropping funnel, a thermometer, a nitrogen gas inlet tube, and an agitator, 200 parts by weight of distilled water was charged. The following liquid was charged into the dropping funnel: a monomer mixture liquid containing 47 parts by weight of N-methacryloyloxyethyl-N,N,N-trimethylammonium chloride (DMC) serving as a cationic vinyl monomer (A), 40 parts by weight of 2,3-dihydroxypropyl (meth)acrylate (GLM) serving as a nonionic vinyl monomer (B-II), 13 parts by weight of N-(2-hydroxyethyl) acrylamide (HEAA) serving as a nonionic vinyl monomer (B-III), and 10 parts by weight of distilled water. After being subjected to nitrogen substitution, the reactor was heated to 90°C. After 0.5 parts by weight of 2,2'-azobis(2-methyl-N-(2-hydroxyethyl)-priopionamide) was charged into the reactor, the monomer mixture liquid was added dropwise to the reactor from the dropping funnel over 4 hours. Reaction was carried out at 90°C for 20 hours after the end of dropwise addition, followed by cooling, whereby a cationic polymer A/B was obtained.

In the obtained cationic polymer A/B, the ratio of constitutional units corresponding to the monomers in all the constitutional units was DMC/GLM/HEAA = 47/40/13 (weight ratio), the weight-average molecular weight was 325,000, the viscosity of a 20 weight percent aqueous solution was 645 mPa·s, the amount of cationic groups was 1.93 meq/g, and the amount of hydroxyl groups was 5.78 meq/g. The weight-average molecular weight and viscosity of the cationic polymer A/B were measured as described below.

### (Weight-average molecular weight)

The weight-average molecular weight was determined by gel permeation chromatography (devices: SC8010, SD8022, RI8020, CO8011, and PS8010 manufactured by Tosoh Corporation; a column: Wakopak (Wakobeads G-50) manufactured by Wako Pure Chemical Industries, Ltd.; and an eluent: water/methanol/acetic acid/sodium acetate = 6/4/0.3/0.41 (weight ratio)) using polyethylene glycol as a standard substance.

### (Viscosity)

A 20 weight percent aqueous solution of the cationic polymer A/B was measured at 30 rpm and 25°C using a B-type viscometer and a No. 2 rotor.

### Production Example 2: Preparation of Dispersion Precursor 1

To a vessel of a batch-type mill ("Ready Mill RMB-02" manufactured by AIMEX Co., Ltd.), 48 g of a powder of ZPT "ZPT-100", 60 g of the cationic polymer A/B (an aqueous solution, produced in Production Example 1, having a solid content of 40% by weight), and 12 g of distilled water were added and further about 223 g of 0.5 mm zirconia beads were added. A dedicated rotor was set to the batch-type mill and grinding was performed at a rotational speed of 1,701 rpm (a peripheral speed of about 4 m/s) for about 16 hours. Dilution and washing were performed by adding distilled water and the zirconia beads were separated, whereby 145.9 g of a dispersion precursor of ZPT was obtained.
The particle size distribution of the obtained PZT dispersion precursor was measured with "Microtrac MT3300EX" manufactured by Nikkiso Co., Ltd., resulting in D₅₀ = 0.400 µm and D₉₅ = 1.082 µm.
The content of ZPT in the dispersion precursor was determined by an HPLC-UV method to be 34.4% by weight.
The dispersion precursor is referred to as "Dispersion Precursor 1".

### Production Example 3: Preparation of Dispersion Precursor 2

To a vessel of a batch-type mill ("Ready Mill RMB-02" manufactured by AIMEX Co., Ltd.), 50 g of a powder of ZPT "ZPT-100", 62.5 g of the cationic polymer A/B (an aqueous solution, produced in Production Example 1, having a solid content of 40% by weight), and 7.5 g of distilled water were added and further about 223 g of 0.5 mm zirconia beads were added. A dedicated rotor was set to the batch-type mill and grinding was performed at a rotational speed of 2,549 rpm (a peripheral speed of about 6 m/s) for about 20 hours. Dilution and washing were performed by adding distilled water and the zirconia beads were separated, whereby 135.7 g of a provisional dispersion of ZPT was obtained. Furthermore, likewise, 50 g of ZPT "ZPT-100", 62.5 g of the cationic polymer A/B (an aqueous solution, produced in Production Example 1, having a solid content of 40% by weight), and 7.5 g of the provisional dispersion after grinding described above and the separation of the beads were added and further about 223 g of 0.5 mm zirconia beads were added. The dedicated rotor was set to the batch-type mill and grinding was performed at a rotational speed of 2,549 rpm (a peripheral speed of about 6 m/s) for about 22 hours. Dilution and washing were performed by adding the provisional dispersion after grinding described above and the separation of the beads and the zirconia beads were separated, whereby 289.4 g of a dispersion precursor of ZPT was obtained in total of two-batch grinding.
The particle size distribution of the obtained PZT dispersion precursor was measured with "Microtrac MT3300EX" manufactured by Nikkiso Co., Ltd., resulting in D₅₀ = 0.411 µm and D₉₅ = 1.034 µm.
The content of ZPT in the dispersion precursor was determined by an HPLC-UV method to be 29.0% by weight.
The dispersion precursor is referred to as "Dispersion Precursor 2".

### Production Example 4: Preparation of Dispersion Precursor 3

To a vessel of a batch-type mill ("Ready Mill RMB-02" manufactured by AIMEX Co., Ltd.), 50 g of a powder of ZPT "ZPT-100", 12.5 g of the cationic polymer A/B (an aqueous solution, produced in Production Example 1, having a solid content of 40% by weight), and 37.5 g of distilled water were added and further about 186 g of 0.5 mm zirconia beads were added. A dedicated rotor was set to the batch-type mill and grinding was performed at a rotational speed of 2,124 rpm (a peripheral speed of about 5 m/s) for about 11 hours. Dilution and washing were performed by adding distilled water and the zirconia beads were separated, whereby 84.5 g of a provisional dispersion of ZPT was obtained. Furthermore, likewise, 60 g of the provisional dispersion, 15 g of the cationic polymer A/B (an aqueous solution, produced in Production Example 1, having a solid content of 40% by weight), and 25 g of the provisional dispersion after grinding described above and the separation of the beads were added and further about 186 g of 0.5 mm zirconia beads were added. The dedicated rotor was set to the batch-type mill and grinding was performed at a rotational speed of 2,124 rpm (a peripheral speed of about 5 m/s) for about 16 hours. Dilution and washing were performed by adding the provisional dispersion after grinding described above and the separation of the beads and the zirconia beads were separated, whereby 195.2 g of a dispersion precursor of ZPT was obtained in total of two-batch grinding.
The particle size distribution of the obtained PZT dispersion precursor was measured with "Microtrac MT3300EX" manufactured by Nikkiso Co., Ltd., resulting in D₅₀ = 0.410 µm and D₉₅ = 1.088 µm.
The content of ZPT in the dispersion precursor was determined by an HPLC-UV method to be 49.8% by weight.
The dispersion precursor is referred to as "Dispersion Precursor 3".

### Production Example 5: Preparation of Dispersion Precursor 4

To an about 200-ml wide-mouth glass jar, 50 g of a powder of ZPT "ZPT-100", 2.5 g of the cationic polymer A/B (an aqueous solution, produced in Production Example 1, having a solid content of 40% by weight), and 72.5 g of distilled water were added and further about 188 g of 0.7 to 1 mm glass beads were added. A stirring rod equipped with three turbine blades with a diameter of 80 mm was set and grinding was performed at 500 rpm to 600 rpm for about 13 hours. The glass beads were separated, whereby 59.7 g of a dispersion precursor of ZPT was obtained.
The particle size distribution of the obtained PZT dispersion precursor was measured with "Microtrac MT3300EX" manufactured by Nikkiso Co., Ltd., resulting in D₅₀ = 0.415 µm and D₉₅ = 1.238 µm.
The content of ZPT in the dispersion precursor was determined by an HPLC-UV method to be 39.3% by weight.
The cationic polymer A/B (an aqueous solution, produced in Production Example 1, having a solid content of 40% by weight) was added to the dispersion precursor, followed by mixing with a vibrating mixer, whereby a ZPT dispersion precursor with an arbitrary ZPT-to-cationic polymer ratio was prepared.
This dispersion precursor is referred to as "Dispersion Precursor 4".

### Production Example 6: Preparation of Dispersion Precursor 5

To a 100-ml glass beaker, 12.5 g of a powder of ZPT "ZPT-100", 0.25 g of polyvinylpyrrolidone (PVP K30) (produced by ISP), and 18.5 g of distilled water were added and further about 40 g of 0.7 to 1 mm glass beads were added. A stirring rod equipped with two turbine blades with a diameter of 40 mm was set and grinding was performed at 1,100 rpm for about 10 hours. The glass beads were separated, whereby 21.5 g of a dispersion precursor of ZPT was obtained.
The particle size distribution of the obtained PZT dispersion precursor was measured with "Microtrac MT3300EX" manufactured by Nikkiso Co., Ltd., resulting in D₅₀ = 0.504 µm and D₉₅ = 1.048 µm.
The content of ZPT in the dispersion precursor was determined by an HPLC-UV method to be 32.6% by weight.
The cationic polymer A/B (an aqueous solution, produced in Production Example 1, having a solid content of 40% by weight) was added to the dispersion precursor, followed by mixing with a vibrating mixer, whereby a ZPT dispersion precursor with an arbitrary ZPT-to-cationic polymer ratio was prepared.
This dispersion precursor is referred to as "Dispersion Precursor 5".

### [Examples 1 to 28]

Surfactants shown in Tables 1, 2, and 3 were added to ZPT dispersion precursors obtained by a method similar to a method used to prepare Dispersion Precursor 1 in Production Example 2 or by a method in which the blending ratio of the cationic polymer A/B was varied in the method used to prepare Dispersion Precursor 1 as shown in Table 3, followed by adjusting the concentrations of ZPT and the cationic polymer A/B, whereby various ZPT dispersions were obtained. The contents of ZPT, the cationic polymer A/B, and the surfactants in the obtained dispersions are as shown in Tables 1, 2, and 3.

### [Comparative Example 1]

The concentration of ZPT in a ZPT dispersion precursor obtained by a method similar to the method used to prepare Dispersion Precursor 1 in Production Example 2 was adjusted without adding any surfactant thereto, whereby a ZPT dispersion was obtained. The contents of ZPT and the cationic polymer A/B in the obtained dispersion are as shown in Table 1.

### [Example 29]

After 3.75 g of EMULGEN 420 (polyoxyethylene (13) oleyl ether produced by Kao Corporation, HLB 13.6), which is a nonionic surfactant, was added to 50 g of a 50 weight percent aqueous dispersion (ZPT-50) of ZPT, 31.25 g of the cationic polymer A/B (an aqueous solution, produced in Production Example 1, having a solid content of 40% by weight) and 15 g of distilled water were added thereto, whereby the concentration of ZPT was adjusted to 25% by weight. The content of ZPT in an obtained dispersion was 25% by weight, the content of the nonionic surfactant therein was 3.75% by weight, and the content of cationic polymer A/B therein was 12.5% by weight.

### [Comparative Example 2]

To 10 g of a 50 weight percent aqueous dispersion (ZPT-50) of ZPT, 6.25 g of the cationic polymer A/B (an aqueous solution, produced in Production Example 1, having a solid content of 40% by weight) and 3.75 g of distilled water were added, whereby the concentration of ZPT was adjusted to 25% by weight. However, ZPT was coagulated as it was; hence, no stable dispersion was capable of being obtained.
The content of ZPT in an obtained dispersion was 25% by weight and the content of the cationic polymer A/B therein was 12.5% by weight.

The content of a component in each of dispersions shown in Tables 1, 2, and 3 is a value expressed in terms of solid. For example, for the cationic polymer A/B (an aqueous solution, produced in Production Example 1, having a solid content of 40% by weight), the content of the cationic polymer A/B in each dispersion is shown and water is added to the distilled water side. This applies to the surfactant "YUKAFORMER 204W". Table 4 only shows the amounts of all components actually used. "ZPT-50" and "cationic polymer A/B" are values for solutions and distilled water is an added value.

### [Evaluation of dispersion stability]

### (Effect of suppressing coagulation of ZPT)

In order to evaluate the effect of suppressing the coagulation of ZPT in the ZPT dispersions obtained in Examples 1 to 24, 26, and 29 and Comparative Examples 1 and 2, each dispersion was left stationary in a 50°C constant-temperature bath, the particle size D₅₀ of ZPT was measured ("Microtrac MT3300EX" manufactured by Nikkiso Co., Ltd.), and the change thereof was checked with time. The dispersion was measured for D₅₀ in an early stage (immediately after preparation), after being held at 50°C for one week, and after being held at 50°C for two weeks and the increment of each value was shown with a score of 100 representing D₅₀ in the early stage (immediately after preparation). The coagulation-suppressing effect was evaluated from the increment of D₅₀ obtained after being held at 50°C for two weeks in accordance with standards below.
A: an increase in index being 10% or less of an initial value (very good in coagulation-suppressing effect).
B: an increase in index being more than 10% to 15% or less of an initial value (good in coagulation-suppressing effect).
C: an increase in index being more than 15% to 20% or less of an initial value (effective in suppressing coagulation).
D: an increase in index being more than 20% of an initial value (ZPT is likely to be coagulated due to no coagulation-suppressing effect).

### (Effect of suppressing precipitation of ZPT)

In Examples 8 to 19 and 21 to 28, in order to evaluate the effect (phase separation resistance) of suppressing the precipitation of ZPT in the ZPT dispersions, the proportion of the height (thickness) of a supernatant layer to the whole height of a liquid layer (= (height of supernatant layer) / (whole height of liquid layer) x 100 (%)) was determined during holding at 50°C as described above and the change thereof was checked with time.

The precipitation-suppressing effect was evaluated from a value obtained after being held at 50°C for two weeks in accordance with standards below.
A: 5% or less (very good in precipitation-suppressing effect)
B: more than 5% to 10% (good in precipitation-suppressing effect)
C: more than 10% to 15% (slightly effective in suppressing precipitation)
D: more than 15% (phase separation likely to occur due to no precipitation-suppressing effect)

Results are shown in Tables 1 to 4.

**[Table 1]**

| | | | Examples | | | | | | | Comparative Example |
|---|---|---|---|---|---|---|---|---|---|---|
| | | | 1 | 2 | 3 | 4 | 5 | 6 | 7 | 1 |
| Dispersion blend (weight percent) | ZPT-100 | | 25.00 | 25.00 | 25.00 | 25.00 | 30.00 | 18.00 | 23.20 | 28.50 |
| | Cationic polymer A/B | | 12.50 | 12.50 | 12.50 | 12.50 | 15.00 | 18.00 | 11.80 | 14.25 |
| | Surfactant | EMULGEN 420 | 2.50 | 3.75 | | | | | | |
| | | IONET T-80V | | | 5.00 | 6.25 | | | | |
| | | YUKAFORMER 204W | | | | | 2.00 | | | |
| | | PVP K30 | | | | | | 0.40 | 0.50 | |
| | Distilled water | | 60.00 | 58.75 | 57.50 | 56.25 | 53.00 | 63.60 | 64.70 | 57.25 |
| | Sum | | 100.00 | 100.00 | 100.00 | 100.00 | 100.00 | 100.00 | 100.00 | 100.00 |
| Coagulation-suppressing effect (D₅₀: index) | | Initial value | 100 | 100 | 100 | 100 | 100 | 100 | 100 | 100 |
| | | After held at 50°C for one week | 111 | 111 | 108 | 105 | 104 | 99 | 107 | 116 |
| | | After held at 50°C for two weeks | 114 | 111 | 115 | 114 | 106 | 118 | 116 | 123 |
| | | Evaluation | B | B | B | B | A | C | C | D |

**[Table 2]**

| | | | Examples | | | | | | | | | | |
|---|---|---|---|---|---|---|---|---|---|---|---|---|---|
| | | | 8 | 9 | 10 | 11 | 12 | 13 | 14 | 15 | 16 | 17 | 18 |
| Dispersion blend (weight percent) | ZPT-100 | | 25.00 | 25.00 | 25.00 | 25.00 | 25.00 | 25.00 | 25.00 | 25.00 | 25.00 | 25.00 | 25.00 |
| | Cationic polymer A/B | | 12.50 | 12.50 | 12.50 | 12.50 | 12.50 | 12.50 | 12.50 | 12.50 | 12.50 | 12.50 | 12.50 |
| | Surfactant | EMULGEN 420 | 2.50 | 3.75 | 3.75 | 3.75 | 3.75 | 3.75 | 3.75 | | | | 3.75 |
| | | IONET T-80V | | | | | | | | 5.00 | 5.00 | | |
| | | YUKAFORMER 204W | | | | | | | | | | 0.50 | |
| | | PVP K30 | 0.25 | 0.25 | 0.63 | | | | | | | | |
| | | EMANON 3199V | | | | 0.25 | | | | 1.00 | | 1.00 | |
| | | EMANON 4110 | | | | | 1.50 | | | | | | |
| | | HEC | | | | | | 0.03 | | | 0.50 | | |
| | | PEG1000 | | | | | | | 1.00 | | | | |
| | | DIANOL CDE | | | | | | | | | | | 1.00 |
| | Distilled water | | 59.75 | 58.50 | 58.12 | 58.50 | 57.25 | 58.72 | 57.75 | 56.50 | 57.00 | 61.00 | 57.75 |
| | Sum | | 100.00 | 100.00 | 100.00 | 100.00 | 100.00 | 100.00 | 100.00 | 100.00 | 100.00 | 100.00 | 100.00 |
| Coagulation-suppressing effect (D₅₀: index) | | Initial value | 100 | 100 | 100 | 100 | 100 | 100 | 100 | 100 | 100 | 100 | 100 |
| | | After held at 50°C for one week | 114 | 116 | 106 | 116 | 115 | 106 | 115 | 112 | 105 | 105 | 111 |
| | | After held at 50°C for two weeks | 117 | 120 | 101 | 120 | 118 | 112 | 115 | 114 | 110 | 106 | 113 |
| | | Evaluation | C | C | A | C | C | B | B | B | A | A | B |
| Precipitation-suppressing effect (supernatant layer proportion: %) | | Initial value | 0 | 0 | 0 | 0 | 0 | 0 | 0 | 0 | 0 | 0 | 0 |
| | | After held at 50°C for one week | 3 | 3 | 6.3 | 2 | 12.9 | 8 | 10.3 | 2.9 | 6.1 | 0 | 13.3 |
| | | After held at 50°C for two weeks | 3 | 6.1 | 6.5 | 2 | 12.9 | 12 | 12.8 | 5.9 | 6.1 | 5.9 | 13.3 |
| | | Evaluation | A | B | B | A | C | C | C | B | B | B | C |

**[Table 3]**

| | | | Examples | | | | | | | | | |
|---|---|---|---|---|---|---|---|---|---|---|---|---|
| | | | 19 | 20 | 21 | 22 | 23 | 24 | 25 | 26 | 27 | 28 |
| Dispersion blend (weight percent) | ZPT-100 | | 25.00 | 25.00 | 25.00 | 25.00 | 25.00 | 25.00 | 30.00 | 30.00 | 35.00 | 35.00 |
| | Cationic polymer A/B | | 12.50 | 6.25 | 12.50 | 6.25 | 6.25 | 6.25 | 6.00 | 6.00 | 7.00 | 7.00 |
| | Surfactant | EMULGEN 420 | 3.75 | | | | | | | | | |
| | | IONET T-80V | | 5.00 | 5.00 | 5.00 | 5.00 | 5.00 | 6.00 | 6.00 | 7.00 | 7.00 |
| | | HEC | | 0.50 | | | | | | | | |
| | | SE600 | | | 0.05 | 0.05 | 0.05 | 0.10 | 0.05 | 0.10 | | 0.05 |
| | | PEG6000 | 0.67 | | | | | | | | | |
| | Distilled water | | 58.08 | 63.25 | 57.45 | 63.70 | 63.70 | 63.65 | 57.95 | 57.90 | 51.00 | 50.95 |
| | Sum | | 100 | 100 | 100 | 100 | 100 | 100 | 100 | 100 | 100 | 100 |
| Coagulation-suppressing effect (D₅₀: index) | | Initial value | 100 | 100 | 100 | 100 | 100 | 100 | - | 100 | - | - |
| | | After held at 50°C for one week | - | 112 | - | - | - | - | - | - | - | - |
| | | After held at 50°C for two weeks | 105 | 114 | 101 | 105 | 107 | 106 | - | 108 | - | - |
| | | Evaluation | A | B | A | A | A | A | - | A | - | - |
| Precipitation-suppressing effect (supernatant layer proportion: %) | | Initial value | 0 | - | 0 | 0 | 0 | 0 | 0 | 0 | 0 | 0 |
| | | After held at 50°C for one week | - | - | - | - | - | - | - | - | - | - |
| | | After held at 50°C for two weeks | 0.9 | - | 0.8 | 3.4 | 7.5 | 8.7 | 3.5 | 3.5 | 2.7 | 5.4 |
| | | Evaluation | A | - | A | A | B | B | A | A | A | B |

**[Table 4]**

| | | | Example29 | Comparative Example2 |
|---|---|---|---|---|
| Dispersion blend (g) | ZPT-50 | | 50.00 | 50.00 |
| | Cationic polymer A/B | | 31.25 | 31.25 |
| | Surfactant | EMULGEN 420 | 3.75 | - |
| | Distilled water | | 15.00 | 18.75 |
| | Sum | | 100.00 | 100.00 |
| Coagulation-suppressing effect (D₅₀: index, data between parentheses is the value of D₅₀ (*µ*m)) | | Initial vale | 100 (0.804) | 100 (12.84) |
| | | After held at 50°C for one week | 97 (0.777) | - |
| | | After held at 50°C for two weeks | 96 (0.768) | - |
| | | Evaluation | A | D (Initial coagulation occurred) |

From Tables 1 to 4, it is clear that an aqueous dispersion of ZPT can be obtained by blending a non-anionic surfactant, the aqueous dispersion being stable even though ZPT is present with a cationic polymer.

While the present invention has been described in detail with reference to specific embodiments, it is apparent for those skilled in the art that various modifications can be made without departing from the spirit and scope of the present invention.
This application is based on a Japanese patent application (Japanese Patent Application No. 2010-183745) filed on August 19, 2010 and a Japanese patent application (Japanese Patent Application No. 2010-284772) filed on December 21, 2010, the entirety of which is incorporated herein by reference.

## Claims

1. An antibacterial dispersion **characterized by** containing at least one cationic polymer, at least one metal pyrithione salt, and at least one non-anionic surfactant.

2. The antibacterial dispersion according to Claim 1, wherein the non-anionic surfactant is a nonionic surfactant and/or an amphoteric surfactant.

3. The antibacterial dispersion according to Claim 2, wherein the nonionic surfactant contains one or more selected from the group consisting of polyoxyethylene alkyl ethers, polyoxyethylene alkylene derivatives, polyoxyethylene fatty acid esters, polyoxyethylene sorbitan fatty acid esters, polyoxyethylene sorbitol fatty acid esters, polyethylene glycols, alkyl alkanol amides, and nonionic water-soluble polymers.

4. The antibacterial dispersion according to Claim 3, wherein the HLB of the nonionic surfactant ranges from 10 to 20.

5. The antibacterial dispersion according to any one of Claims 1 to 4, wherein the metal pyrithione salt is a salt of any one of Zn, Cu, Fe, Al, and Mg.

6. The antibacterial dispersion according to Claim 5, wherein the metal pyrithione salt is a Zn salt.

7. The antibacterial dispersion according to any one of Claims 1 to 6, wherein the cationic polymer is a hair conditioner.

8. The antibacterial dispersion according to Claim 7, wherein the cationic polymer is a copolymer which contains constitutional units corresponding to a cationic vinyl monomer (A) represented by the following formula (I) and constitutional units corresponding to nonionic vinyl monomers (B) represented by the following formulae (II) and/or (III), in which the proportion of the constitutional units corresponding to the cationic vinyl monomer (A) to the total constitutional units is 10% to 80% by weight, and which has a weight-average molecular weight of 10,000 to 2,000,000:
CH₂=C (R¹) -CO (O)ₐ- (NH)₁₋ₐ- (CH₂)_{b}-N⁺R²R³R⁴·X⁻ (I)
(where R¹ represents a hydrogen atom or a methyl group; R² and R³ independently represent an alkyl group containing one to 24 carbon atoms, an aryl group containing one to 24 carbon atoms, or an aralkyl group containing one to 24 carbon atoms; R⁴ represents a hydrogen atom, an alkyl group containing one to 24 carbon atoms, an aryl group containing one to 24 carbon atoms, an aralkyl group containing one to 24 carbon atoms, or -CH₂-CH(OH)-CH₂N⁺R⁵R⁶R⁷·Y⁻; R⁵, R⁶, and R⁷ independently represent an alkyl group containing one to 24 carbon atoms, an aryl group containing one to 24 carbon atoms, or an aralkyl group containing one to 24 carbon atoms; X⁻ and Y⁻ independently represent an anion; a represents 0 or 1; and b represents an integer of 1 to 10),
CH₂=C (R⁸) -CO (O) -Z-H (II)
(where R⁸ represents a hydrogen atom or a methyl group and Z represents a bivalent linker containing two or more hydroxyl groups),
CH₂=C (R⁹) -CO-NR¹⁰R¹¹ (III)
(where R⁹ represents a hydrogen atom or a methyl group and R¹⁰ and R¹¹ independently represent a hydrogen atom, an alkyl group containing one to four carbon atoms, or a hydroxyalkyl group containing one to four carbon atoms).

9. The antibacterial dispersion according to any one of Claims 1 to 8, wherein the blending ratio of the pyrithione salt to the cationic polymer is 1/1 to 50/1 on a weight basis.

10. The antibacterial dispersion according to any one of Claims 1 to 9, wherein the content of the metal pyrithione salt is 5% to 70% by weight, the content of the cationic polymer is 0.1% to 40% by weight, and the content of the non-anionic surfactant is 0.01% to 25% by weight.

11. The antibacterial dispersion according to any one of Claims 1 to 10, wherein the ratio of the content of the metal pyrithione salt to the content of the non-anionic surfactant is such that the pyrithione salt/non-anionic surfactant ratio is 10,000/1 to 1/1 on a weight basis.

12. The antibacterial dispersion according to any one of Claims 1 to 11, being produced from the following precursor:
(i) a dispersion precursor which is obtained by grinding and mixing a powder of the metal pyrithione salt, the cationic polymer, and water and which has a metal pyrithione salt concentration of 30% to 80% by weight or
(ii) a dispersion precursor which is obtained by grinding and mixing a powder of the metal pyrithione salt, the non-anionic surfactant, the cationic polymer, and water and which has a metal pyrithione salt concentration of 30% to 80% by weight.
